# EUROPEAN PATENT APPLICATION

(11) **EP 3 338 544 A1**
(43) Date of publication of application: **27.06.2018**
(21) Application number: 16836298.6
(22) Date of filing: 16.08.2016
(51) Int. Cl.: A01K 45/00

(54) **IMPROVEMENT TO FUNCTIONAL MODULE FOR THE SELECTIVE APPLICATION OF SUBSTANCES INSIDE FERTILE EGGS**

(30) Priority: 18.08.2015 BR 102015019877
(71) Applicant: Ceva Saúde Animal Ltda., Santa Terezinha, Paulínia SP (BR)
(72) Inventor: Bastos, César da Silva, 13020-060 Campinas - SP (BR)
(74) Representative: Cabinet Becker et Associés
(86) International application number: PCT/BR2016/050194
(87) International publication number: WO 2017/027947

(57) **Abstract**

An improvement to a functional module for the selective application of substances inside fertile eggs represents an inventive solution in the field of poultry farming, in particular in the field of poultry breeding, and more specifically in the step of application of vaccines and/or nutrients into fertile eggs by injection, this improvement reducing the risk of violation of biological safety, and ensuring the integrity and survival of the embryo (Em), limiting *intra-ovum* nutritional supplementation to the amniotic fluid (Li) only, without contact of the needle (al3) with the embryo (Em), and to achieve this condition the module for the selective application of substances inside fertile eggs (Ov) has a structure composed of an injector selector submodule (ml), a selective operation actuation submodule (m2), a speed control submodule (m3) and a common perforator/needle return submodule (m4).

## Description

### TERMINOLOGY

For a better understanding of the subject matter disclosed and claimed in the present patent application, the meaning of some terms richly cited in the body of the descriptive report is shown, in which:

- Egg-candling: it is a procedure to verify if the eggs are actually fertilized (fertile) and if they are not, they are removed from the process of raising poultry or the like.

### APPLICATION FIELD

The present invention patent of title in the epigraph and subject of description and claim in this application deals with an inventive solution that gives an outstanding benefit to the poultry segment, notably in the sector of poultry reproduction, and more specifically in the stage of application of vaccines and or nutrients in fertile eggs by injection.

### BACKGROUND OF THE INVENTION

In view of the field of application, the applicant, an expert in the area of breeding and development of embryos for breeding of chickens, after detailed studies of the entire classification, vaccination and/or nutrition and hatching techniques, identified a list of emerging needs at the vaccination stage and or nutrition:
- optimization of the productivity of vaccinated fertile eggs and or with nutritional supplement;
- needs to guarantee the biological safety condition, resulting in the elimination of the risk of contamination of the substance itself (vaccine and nutritional supplements);
- providing a condition for controlling the parameter of penetration speed of the injection needle inside the fertile egg, which varies according to the type of substance to be inoculated (vaccines or nutrients, for example); and
- minimizing losses by breaking the shells of the eggs during the interference of the egg with the module of application of substances inside fertile eggs.

### REQUIREMENTS FOR THE INVENTION

In accordance with the demand of the invention the applicant conceived the *"IMPROVEMENT APPLIED TO THE FUNCTIONAL MODULE FOR SELECTIVE APPLICATION OF SUBSTANCES INSIDE FERTILE EGGS"* provided with novelty associated with inventive activity, since it does not occur in an obvious or evident way from other techniques anticipated by the state of the art, conferring advantages from the industrial, commercial and technical points of view.

In addition, the *"invention"* is provided with industrial applicability, being economically feasible and thus, considering the accuracy of the patentability requirements, mainly an invention patent, as set forth in the dictates of articles 8 and 13 of Law 9.279.

### BACKGROUND OF THE TECHNIQUE

In order to provide veracity and to consolidate the explicit context in the topics of the introductory table, an explanation will be presented on the state of the art in conventional vaccination modules of fertile eggs, whereby after a critical analysis of these, once evaluated by experts in the subject, can identify its limiting aspects, thus consolidating the identification of the previously cited claim.
a. Architecture of the conventional system: in tight synthesis, selection, vaccination of fertile eggs and dispatch for incubation are carried out by a modular, non-integrated system, that is, the poultry farmer can make use of all or only part of its operational modules, being that for the acculturation, the said conventional system is formed by the following operating modules which are briefly described in the following paragraphs.
   a.1 Identification module for fertile eggs: by which, in closed understanding, the operation of selecting fertile eggs can be done with equipment of a manual or automated nature. For equipment of manual nature, the selection operation is done with the aid of a table that emits light under the eggs and the operator visualizes those that allow a greater passage of light classifying them as infertile eggs. In automated systems, there are sensors provided with the emitter and receiver of the light that passes through the eggs. In both situations, a human-machine interface (HMI) management subsystem, or alternatively a handheld equipment having only one table with a light-emitting source is defined, this subsystem acting on the incubation tray, identifying the niches provided with "fertile eggs" and "non-fertile eggs".
      In this way, the procedure of removal of the infertile eggs from the incubation tray takes place, being this operation carried out through a platform with suction cups, and only the "fertile eggs" being kept in the incubation tray".
   a.2 Application module for substances in fertile eggs: where vaccination and or nutrition are performed through injector devices provided with punches and needles for each egg housing niche defined in the incubation tray. From the operational point of view, once the incubation tray is positioned under the vaccination module, the injector array is driven down collectively by moving towards the corresponding incubator tray nodes, where at that time, a computerized system released a vaccine dose (or nutrients) to the needles; and
   a.3 Incubation module for fertile eggs: after vaccination of the fertile eggs contained in the incubator tray, these fertile eggs are transferred into a basket, said delivery basket, the transfer being carried out by means of an array of suction cups disposed in a matrix form, which descend simultaneously, where the vacuum is applied both to the suction cups corresponding to the fertile eggs and to the empty housings. The fertile eggs accommodated in the incubator tray are "sucked" by the suction cups, and through a vertical movement, they are transferred to the breeder basket.
b. The paradigm of the invention: once the architecture of the identification system, fertile egg vaccination and incubation of fertile eggs have been presented in general, for the purpose of the present invention has chosen the module of vaccination and/or nutrition of fertile eggs.
   Patent database research reveals the prior art vaccination modules, where the following patent documents deserve special mention:
   - U.S. Patent No. 6,286,455, entitled "AUTOMATED IN OVO INJECTION APPARATUS", published 11/09/2001, which discloses a module of simultaneous application of multiple substances by injection applied to eggs originating preferentially from the egg-candling stage.
   - US Pat. No. 8,025,028, entitled "AUTOMATED EGG INJECTION MACHINE AND METHOD", published on 27/09/2011, discloses a module for simultaneous application of multiple substances by injection applied to eggs originating preferably from the egg-candling step, and also it shows in detail, the constructive and operational concept of the device for application of the multiple substances.

   Prior art documents have been shown only for the purpose of prior knowledge that the modules for the application of substances to fertile eggs have long been known, in which the most commonly applied substances are vaccines, and, more recently, the application of nutrients have been considered.
   In an allusive way, the constructive concept of the substance application module in fertile eggs is composed of an accommodation and transport device, including a tray in which egg accommodation niches as defined in a matrix form, by which this tray is positioned immediately below the device of multiple injectors of substances, each positioned immediately above its respective egg accommodation niche.
   Also, in an allusive way, each injector device has the function of opening the access to the interior of the egg by means of a shell opening mechanism and an injection needle in the most varied configurations possible.
   In turn, from the operational point of view, once the niche parity - injector device condition is established, the substance application module is actuated in such a way that simultaneously all the injecting devices are activated, causing the perforation mechanisms of the eggshell, and subsequently the injection needle, which is the carrier element of the substance inside the fertile egg.
   In addition to being of significant importance, it should be made clear that the substance to be inoculated with each of the injection needles from each injection device comes from a single source, i.e., a single reservoir, which is released to branches corresponding to each injection device.
c. Critical analysis of the vaccination module and or nutrition: although the current modules for the application of multiple substances into fertile eggs are effective with regard to the procedure of injection of fertile eggs, the careful observation of experts in the area of fertilization and development of poultry embryos identified a list of problems on which should be highlighted:
   c.1 From the biosafety point of view: it is explicit the risk of contamination of the injected substance leading to the compromise of the whole stored volume, thus imposing the disposal and sterilization procedure of the entire transport circuit of the substance, from the reservoir to the injection needle;
   c.2 From an application level adjustment point of view: where it is explicit that there is only one level of penetration of the needle inside the fertile egg, and no distinction is made on the type of substance being inoculated; and
   c.3 from the productivity point of view: the procedure of approximation and contact of the injection device with the fertile eggs, invariably generates some productivity losses by the breaking of eggshell, and the discard of fertilized eggs is being forced.
d. Why do problems occur (cause of effects):
   d.1 From the bio-security point of view: can also be explained by the fact that the substance application module is not selective, that is, it does not recognize niches where there is no presence of fertile eggs, due to the process of exclusion of non-fertile eggs carried out in the egg-candling stage, which causes the entire array of injection devices to be triggered, releasing substance even in the area where the niche is empty.
      In this scenario, the exposure of the injection needle to the environment becomes evident, which does not occur with the injection needles that effectively inoculated the interior of the eggs.
   d.2 From an application level adjustment point of view: it can be explained by the fact that until recently, the substance effectively inoculated was the vaccine, which as a rule, counted on the injection devices originally developed under well-defined inoculation conditions in relation to the amniotic fluid and the embryo itself, and there was no need for control.
      However, with the advent of the increasing frequency of practice of nutritional supplementation of fertile eggs, need to control the level of advancement of the injection needle was created, as for this type of nutritional complement, the substance must be poured into the amniotic fluid and not into the embryo's body.
   d.3 From the productivity point of view: the cause of the eventual breaks of the eggs after contact of the injection devices can be explained as a result of the constructive concept of the components: head, body and plunger, manufactured on metallic material whose weight contributes to the occurrence of shell break index.

### DESCRIPTION OF THE INVENTION

a. Objective: it is an object of the present invention to solve the causes of the problems previously listed in the topic of the fundamentals of the art, by showing an improvement in the application module of substances in the interior of fertile eggs, which adds value in relation to the conventional module, by which in an allusive way this improvement confers the following predicates:
   - optimizing the consumption of compressed air during the operation of the substance application module;
   - giving priority to biosafety of the operator module, eliminating the risk of contamination of the substance to be inoculated by undue exposure of needles in areas where no fertile egg is present, i.e. in areas where the tray niche is empty;
   - providing a better control of the penetration speed of the injection needle inside the egg, allowing variations when the circumstances require needle interference in the embryo's body, or only within the volume of the amniotic fluid, in case the substance is a nutrient;
   - minimizing losses by eggshell rupture as caused by the interference of the handle mechanism and stability of the injection device.
b. Distinctive feature: to make possible the predicates of the improvement in a module of selective application of substances in fertile eggs, an unprecedented system of selective activation of devices of injection of substances has been designed, that is, only the injection devices in which a fertile egg is recognized in the tray niche will be activated.

For this purpose, each substance injection device comprises an individual needle feed module, in the form of a 3/2-way valve which acts in the advanced procedure of its injection needle out of the egg.

In turn, this 3/2-way valve is only activated when the selective injection system of substances identifies the respective tray niche with the fertile egg.

In addition, by looking at a situation when the nutrient administration procedure is a needle feed rate control module, in the form of a feed rate control valve, required for the single procedure of inoculation of the substance into the amniotic fluid, where this speed must be such as to prevent the perforation of the embryo by the needle bevel, in case of incidental contact with its epidermis.

Finally, to meet the goal of minimizing the loss by rupturing the eggshell during the procedure of contacting with the injector, the contact components, notably the head, body and injector piston which are manufactured by 3D forming technology (3d printer) by the process of adding molten ABS filaments, by which this process enables a component which is about 50% lighter.

### DESCRIPTION OF THE FIGURES

To complement the present description in order to obtain a better understanding of the features of the present invention, and according to a preferred practical embodiment thereof, the description is accompanied by a set of appended drawings, wherein:
- Fig. 1 is an illustrative representation in perspective of the constructive concept of the module for the application of substances to fertile eggs anticipated by the state of the art, with reference to Fig. 2 of U.S. Patent No. 6,286,455, highlighting the main component parts;
- Figs. 2a and 2b are an illustrative representation inside view of the module concept for the application of substances in fertile eggs, as anticipated by the state of the art, showing the beginning of the operative procedure of injection of substances into a matrix formed by the accommodation tray of fertile eggs, considering that there are niches with absence of eggs, immediately before and after their activation respectively, revealing the problem of biological safety risk and unnecessary injection of substances;
- Fig. 3 is an illustrative side view of the constructive concept of the fertile egg application module anticipated by the state of the art, with reference to Fig. 1 of U.S. Patent No. 6,286,455, showing the main components as well as the logic of its operational drive, evidencing the problem of the collective activation of all injection devices of the device matrix;
- Fig. 4 is an illustrative side view of the constructive concept of the fertile egg application module anticipated by the state of the art, with reference to Fig. 3 of U.S. Patent No. 6,286,455, showing the main components as well as the logic of its operational drive, evidencing the problem of the collective activation of all injection devices of the device matrix;
- Figs. 5a and 5b are illustrative in side view representations of the concept of the improved application module of substances in fertile eggs, showing the beginning of the operative procedure of injecting substances into a matrix formed by a tray of fertile eggs, in which there are niches with absence of eggs immediately before and after their activation respectively, and revealing the problem of biological safety risk and unnecessary injection of substances;
- Fig. 6a is an illustrative front view of the differential injection device for substances, showing its external component parts;
- Fig. 6b is an illustrative representation in the rear view of the differential injection device for substances, showing its external component parts; Fig. 6c is an illustrative cross-sectional view of the differential injection device for substances, showing its external and internal component parts;
- Fig. 7a is an illustrative representation of the individual activation subsystems of the individual displacement and velocity control for each needle as well as the collective retraction subsystem of the needle matrix;
- Fig. 7b is an illustrative representation of component parts of the individual activation subsystems and of individual displacement velocity control for each needle as well as the collective return subsystem of the needle matrix;
- Fig. 8a is an illustrative illustration of the needle feed operation of a conventional injection device, useful for injection of vaccines, but not indicated for the inoculation of nutrients, showing that in this condition, penetration of the needle bevel occurs in the body of the embryo;
- Fig. 8b is a graphical representation showing the evolution of approaching velocity: from the components of the collective needle valve assembly, from the support platform of the injector matrix and needle feed cylinder to the conventional needle injection, suitable for the injection of vaccines;
- Fig. 9a is a graphical representation of the evolution of approach velocity: from the components of the individual needle valve, punch feed cylinder and the needle feed cylinder, to each injection device of the matrix of devices, suitable for inoculation of vaccines and nutrients; and
- Fig. 9b is an illustrative representation of the needle feed operation of an improved injection device, useful for inoculating vaccines and nutrients into the amniotic fluid, showing that, in this condition, there is no penetration of the needle bevel into the body of the embryo.

### DETAILED DESCRIPTION

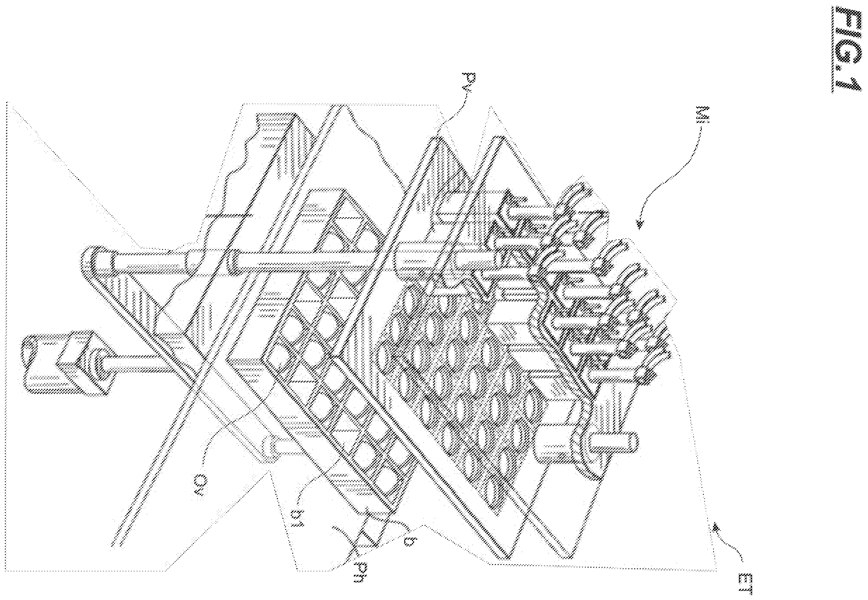

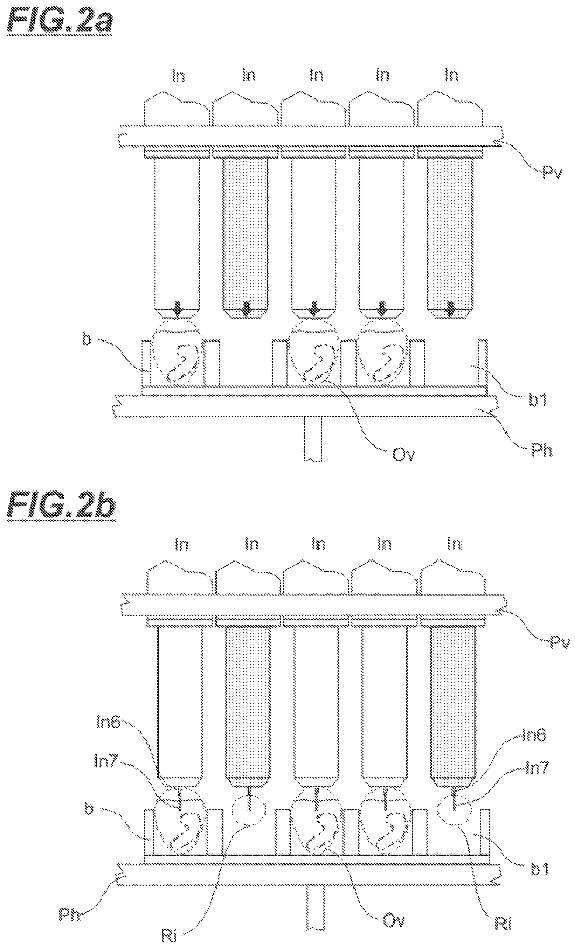

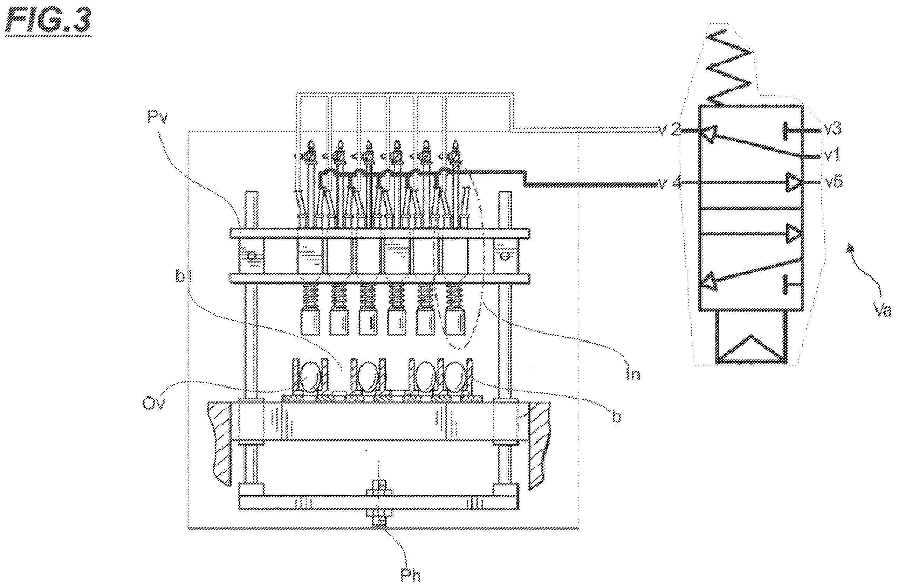

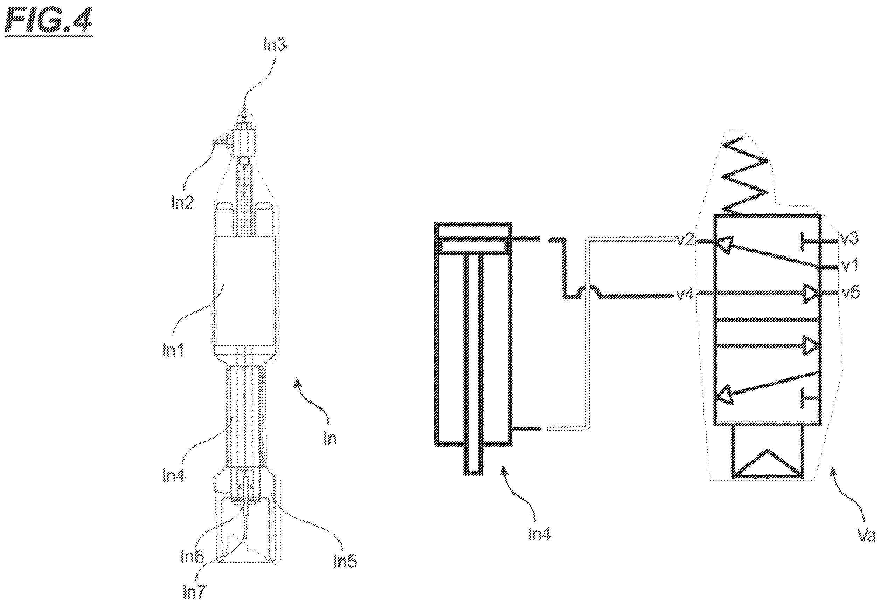

The following detailed description should be read and interpreted with reference to the presented drawings, not intended to limit the scope of the invention, this being limited only as explicit in the content of the claims.
a.1 State of the art:
   As shown in Fig. 1, the conventional fertilizing egg substance (Et) module is composed essentially of the following devices:
   - horizontal displacement platform (Ph): has the function of providing the displacement and positioning of the tray (b) in a way aligned with the matrix of injector devices (d);
   - tray (b), provided with a plurality of niches (b1) in which the fertile eggs (Ov) are accommodated;
   - vertical displacement platform (Pv): it has the function to provide the assembly and stabilization of the injector matrix (Mi), also providing its vertical displacement;
   - injection devices (In): with the purpose of providing injection of substances, notably vaccines, consisting basically of an input for needle advancement (In7) and another for return, being a pneumatic double acting cylinder system (In4), whose actuation is collective for all injectors (In) of the matrix, by means of single collective control valve (Va), see Figs. 3 and 4. The punch (In6) is introduced into the egg (Ob) by mechanical actuation of the vertical displacement (Pv), where the injectors (In) are aligned above the eggs (Ov), see Figs. 2a and .2b. In turn, at the top of each injector (In) is defined a substance access connector (In3) and a connector for sanitizing the access of the substance (In2);

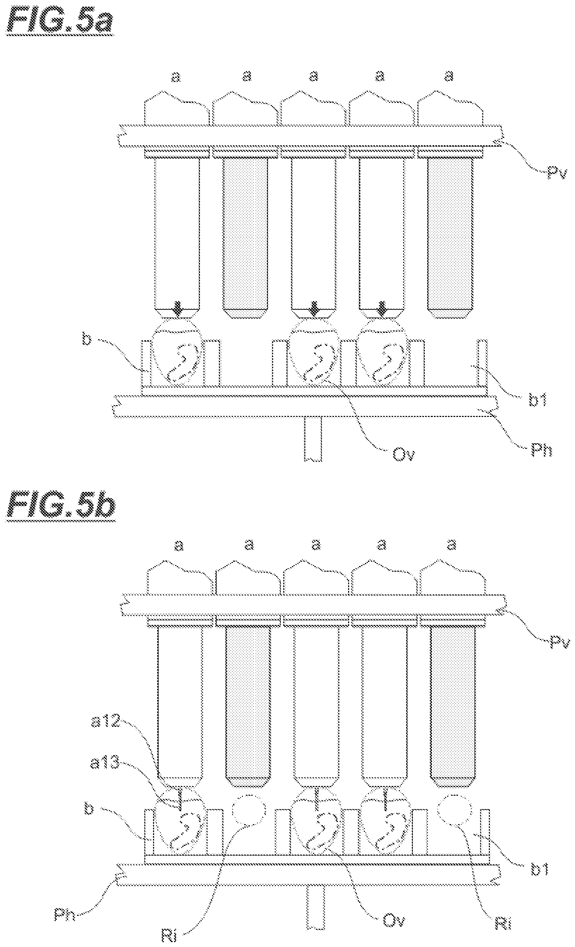

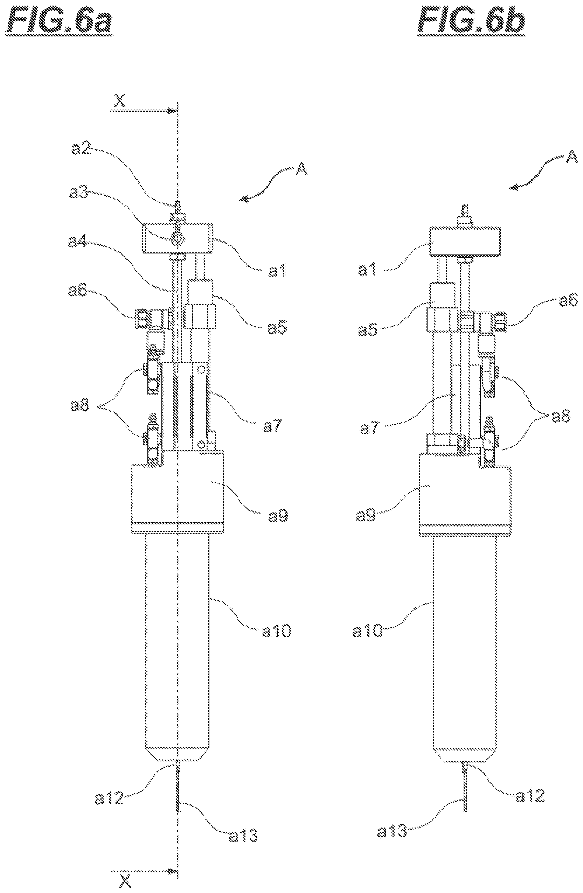

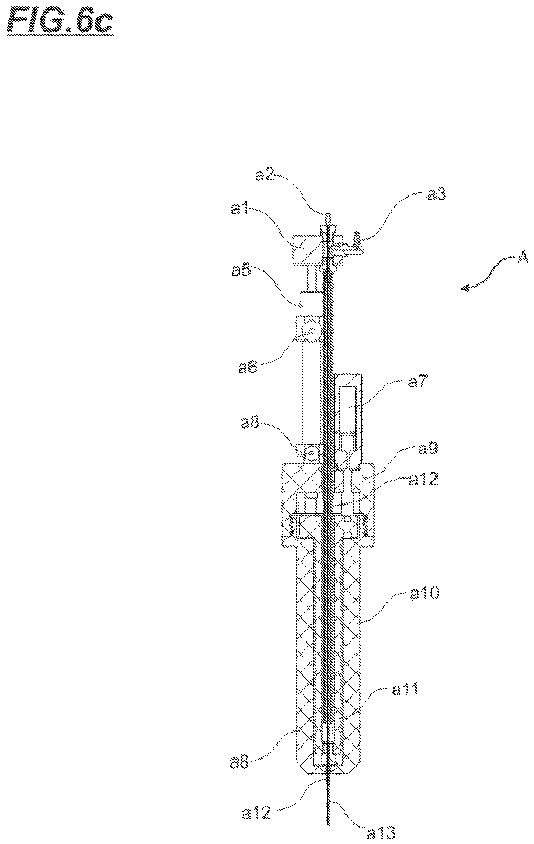

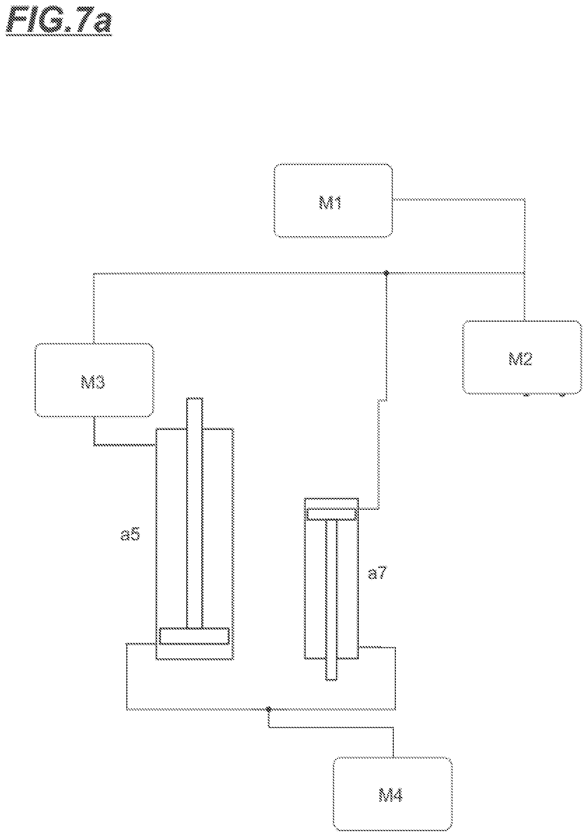

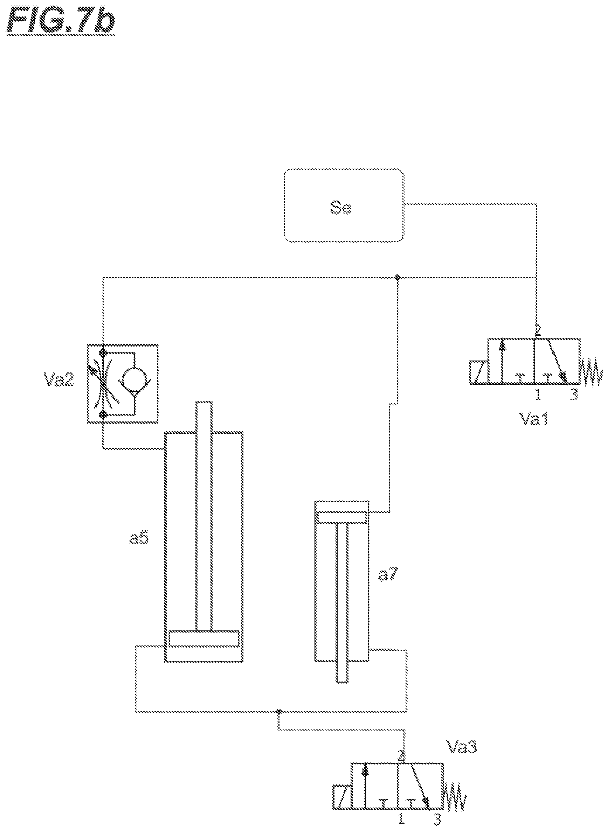

   The punch (In6) and needle end (In7) components, when in non-operational condition, are collected inside the casing or head (In5);
a.2 Evidenced problem: as shown in Figs. 2a and 2b, in operative condition, all needles (In7) are driven in vertical movement away from the head (In5), where effectively independent of the presence of fertile eggs (Ov) in the niches (b1) of the tray (b), becoming evident the exposure to the external environment of the ends of the needles (In7) in the niches (b1) with absence of fertile eggs (Ov), converging to a biological safety risk, since this exposure can lead to the contamination of the substance retained in the needle (In7) that migrates to the substance reservoir, thus generating loss and financial loss.
a.3 Cause of the problem: the scenario described above only occurs because of the constructive concept of collective activation of the downward vertical movement of the needles, which is controlled by a collective 5/2-way valve (Va), where a connection (v4) is provided which activates the forward signal of the needles (In7) of each injector (In), and a connection (v2) which activates the needle retraction signal (v2) of each injector (In) of the injector matrix.
b. About the improved substance application module: it has been designed so that the objects of the invention are practicable to actuate the needles only from the injectors (A) having a corresponding fertile egg (Ov) thereon, as shown in Figs. 5a and 5b, and further in addition that the said forward activation is done at controlled speed, wherein for this purpose the injector matrix is formed by a plurality of improved injectors (A), whose constructive concept is disclosed in Figs. 6a; 6b and 6c, and formed from the functional components:
   b.1 Base technology (provided in the conventional gun):
      - needle coupler (a1);
      - nutrient connector (a2);
      - sanitizing connector (a3);
      - punch feed cylinder (a5);
      - needle feed cylinder (a7);
      - straight pneumatic connection (a8);
      - cover (a9);
      - casing (a10);
      - plunger (a11);
      - M5 punch (a12); and
      - needle (a13).
   b.2: Introduced improvements:
      - Injector selector sub-module (m1): as shown in Figs. 7a and 7b, in the form of a selector device (Se), where it sends a signal to the selective operation actuation sub-module (m2);
      - Selective operation actuation sub-module (m2): as shown in Figs. 7a and 7b, wherein in a preferred embodiment it is specified as a dedicated valve (Va1), notably an individual 3/2-way valve for the advancement of each punch (a12) and respective needle (a13), which is connected to the needle feed cylinder (a7) and punch feed cylinder (a5);
      - Speed control sub-module (m3): as shown in Figs. 7a and 7b, wherein in a preferred embodiment it is specified as a needle speed feed control valve (Va2) acting adjacent to the needle feed cylinder (a7). Effectively, the needle displacement speed (a13) must be controlled to 35 mm per second.
      - Collective punch/needle return sub-module (m4): as shown in Figs. 7a and 7b, wherein in a preferred embodiment it is specified as a collective valve (Va3), notably a collective 3/2-way valve for return of the punch feed cylinders (a5) and the needle feed cylinders (a7) of all injectors (A).

### C. The technical effect obtained:

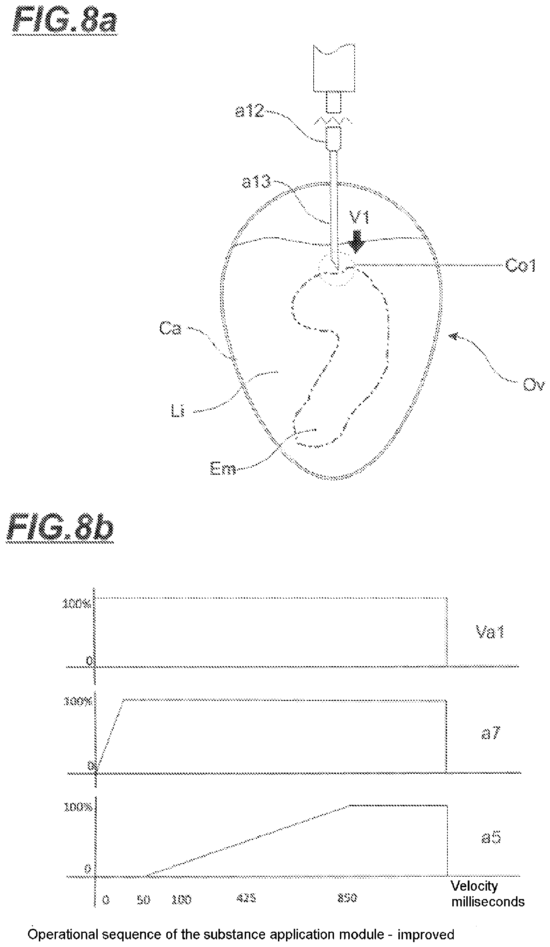

c.1 Selective inoculation of substances: as shown in Figs. 5a and 5b, where only are activated the punch feed cylinder (a5) and the needle feed cylinder (a7) of the injectors (In) of the matrix in which the presence of fertile eggs is identified (Ov) in niche (b1) of tray (b), eliminating undue exposure of needles (a13) by eliminating the risk (Ri) of substance contamination (vaccine, nutrients, among others), see Fig. 5b;
c.2 Preservation of the embryo under nutritional supplementation: as shown in Fig. 8a wherein for nutritional supplementation there is a need to control the feed rate (VI) of the needle (a13) inside the fertile egg (Ov), ensuring a safety condition (Co1), where there is no needle penetration (a13) into the embryo tissue (Em), so that the nutrient is released only in the amniotic fluid (Li). In addition, Fig. 8b shows the obtained feed rate (VI) necessary for the dedicated valve (Va1), needle feed cylinder (a5) and punch feed cylinder (a7), in order to obtain the safety condition (Co1).

Optimum feed rate (VI) should be ≤ 35 mm per second.

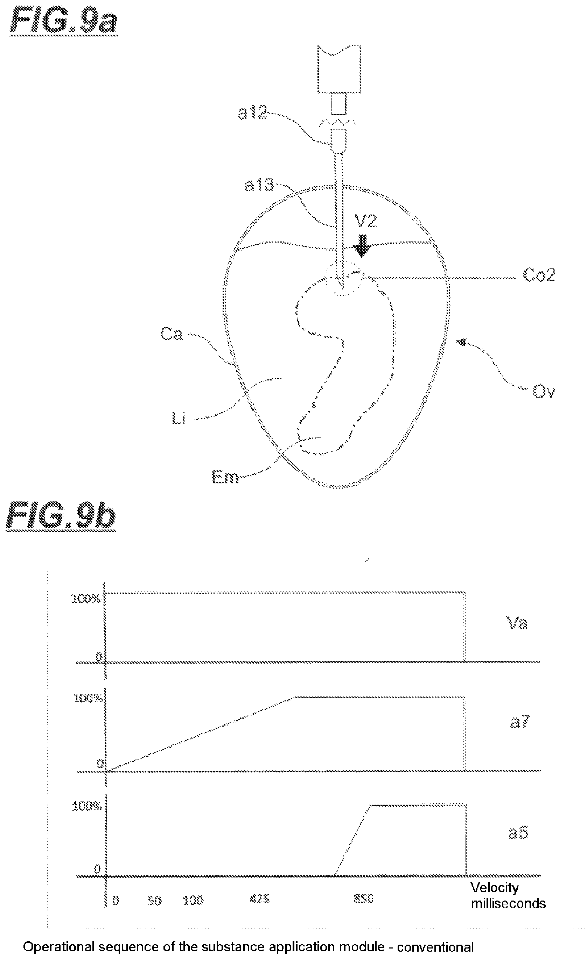

Only for comparative purposes, Fig. 9a shows a condition of a vaccination procedure, where the condition is allowed and desired (Co2) with needle penetration (a13) into the embryo musculature (Em). Fig. 9b shows the feed rate condition (V2) commonly observed in the vaccination procedure, for the collective valve (Va), vertical displacement platform (Pv) and needle feed cylinder (a5).

The choice of the preferred embodiment of the invention claimed in this carton, described in this detailed section, is given by way of example only. Any changes, modifications, and variations may be made to any other embodiments of the improved substance application module and corresponding improved injector device (A), which comprises the injector matrix, wherein changes may be designed by those skilled in the art, however without diverging from the object disclosed in the application of the present patent, which is exclusively defined by the annexed claims.

It is seen from what has been described and illustrated that the "IMPROVEMENT APPLIED TO THE FUNCTIONAL MODULE FOR SELECTIVE APPLICATION OF SUBSTANCES INSIDE FERTILE EGGS" heretofore claimed falls within the rules governing the patent in the light of the Industrial Property Law, thus deserving of what has been stated and as a consequence, the respective privilege.

## Claims

1. Improvement applied to the functional module for selective application of substances inside fertile eggs, wherein a substance application module is formed by a horizontal displacement platform (Ph) which positions a tray (b) with niches (b1) of shape aligned with the matrix of injector devices (Mi), a vertical displacement platform (Pv) that supports the injector matrix (Mi), whose injectors have a technological base formed by a needle coupler (a1), a nutrient connector (a2), a sanitizing connector (a3), a punch feed cylinder (a5), a needle feed cylinder (a7), a straight pneumatic connection (a8), a cover (a9) a casing (a10), a plunger (a11), a M5 punch (a12) and a needle (a13) in which the architecture of the improved substance application module is **characterized in that** it is composed by an injector selector sub-module (m1) which sends signal to the selective operation actuation sub-module (m2) in the form of a dedicated valve (Va1), notably an individual 3/2-way valve connected to the needle feed cylinder (a7) and to the punch feed cylinder (a5), a speed control sub-module (m3) being further defined in the form of a needle speed feed control valve (Va2) acting adjacent to the needle feed cylinder (a7) and further defining a collective punch/needle return sub-module (m4) in the form of a collective valve (Va3), notably a collective 3/2-way valve coupled to the punch feed cylinders (a5) and needle feed cylinders (a7) of all the injector devices (A).

2. Improvement applied to the functional module for selective application of substances inside fertile eggs according to claim 1, wherein once operative, the needle feed cylinder (a7) is **characterized in that** it moves the needle (a13) at an ideal feed rate (VI) that should be ≤ 35 mm per second.
